# EUROPEAN PATENT APPLICATION

(11) **EP 4 819 061 A1**
(43) Date of publication of application: **07.10.2026**
(21) Application number: 26169697.5
(22) Date of filing: 31.03.2026
(51) Int. Cl.: G06V 10/25, G06T 7/00

(54) **X-RAY CT APPARATUS AND IMAGE PROCESSING APPARATUS**

(30) Priority: 31.03.2025 JP 2025058753; 27.03.2026 JP 2026053874
(71) Applicant: Canon Medical Systems Corporation, Tochigi 324-0036 (JP)
(72) Inventor: HORIO, Kyotaro, Otawara-shi (JP); NISHIOKA, Takahiko, Otawara-shi (JP); ANDO, Daiki, Otawara-shi (JP); KUNEZAKI, Haruka, Otawara-shi (JP); KAWASAKA, Sumika, Otawara-shi (JP)
(74) Representative: Marks & Clerk LLP

(57) **Abstract**

An X-ray CT apparatus (200) according to an embodiment includes an X-ray detector (12) and processing circuitry (150). The X-ray detector (12) detects X-rays emitted from an X-ray tube and transmitted through a subject. The processing circuitry (150) generates input data(50) based on data obtained from the X-ray detector (12), when executing inference on a trained model, obtains a feature value from the trained model with respect to a region of interest set based on the input data(50), based on the obtained feature value, determines whether or not to continue the inference with respect to the regions of interest, and stops the inference on determining not to continue the inference and continue the inference on determining to continue the inference.

## Description

### BACKGROUND

When volume data is processed by deep learning, because of a problem of limitation of memory, and the like, in some cases, processing is not performed on the whole range of volume data and the volume data is divided into volumes of interest (VOI) in a fixed size and processing is performed on each divided VOI.

When a VOI size is large, however, processing may take time.

### SUMMARY OF INVNETION

An X-ray CT apparatus that is provided in one aspect of the disclosure includes an X-ray detector and processing circuitry. The X-ray detector detects X-rays emitted from an X-ray tube and transmitted through a subject.

The processing circuitry
generates input data based on data obtained from the X-ray detector,
when executing inference on a trained model, obtains a feature value from the trained model with respect to a region of interest set based on the input data,
based on the obtained feature value, determines whether or not to continue the inference with respect to the region of interest, and
stops the inference on determining not to continue the inference and continue the inference on determining to continue the inference.

An image processing apparatus that is provided in one aspect of the disclosure includes processing circuitry. The processing circuitry
when executing inference on a trained model, obtains a feature value from the trained model with respect to regions of interest set based on input data,
based on the obtained feature value, determines whether or not to continue the inference with respect to the regions of interest, and
stops the inference on determining not to continue the inference and continues the inference on determining to continue the inference.
The regions of interest may be regions of interest into which the input data is divided.

The regions of interest may be regions of interest that correspond to a portion of the input data.

The processing circuitry may obtain feature values for each of the regions of interest into which the input data is divided, and determine whether or not to continue the inference with respect to each of the regions of interest based on the obtained feature values.

The processing circuitry may include a display control unit that causes a display to display a result of determination.

The input data may be two-dimensional or three-dimensional image data, and the regions of interest may be two-dimensional or three-dimensional regions of interest.

The processing circuitry may perform the determination by sequentially performing determination on the feature value in each of a plurality of layers in the trained model.

As for the regions of interest that are selected by a user, the processing circuitry may cause the display unit to display an output of the feature value or a result of the inference such that the output is superimposed onto a result of the determination with respect to each of the regions of interest.

The processing circuitry may receive an input of a change in a threshold of the determination from a user.

The processing circuitry may cause the display to further display a result of the determination in a natural language.

The processing circuitry may cause the display to display a region of interest on which the inference is being executed in distinction from other regions of interest.

The trained model may include a plurality of trained models, and the processing circuitry may cause the display to display information on the feature values of the respective trained models.

The processing circuitry may cause the display to display the feature value such that the feature value is superimposed onto input data.

An image processing apparatus that is provided in one aspect of the disclosure includes processing circuitry. The processing circuitry
when executing training on a trained model, obtains a feature value from the trained model with respect to a region of interest set based on input data,
based on the obtained feature value, determines whether or not to continue training on the trained model with respect to the region of interest,
stops the training with respect to the region of interest on determining not to continue the training with respect to the region of interest and continues the training with respect to the region of interest on determining to continue the training.

According to at least one of the embodiments described above, it is possible to reduce the calculation cost at the time of inference in a neural network.

### BRIEF DESCRIPTION OF THE DRAWINGS

FIG. 1 is a diagram illustrating an example of a configuration of an image processing apparatus according to an embodiment;
FIG. 2 is a diagram illustrating an example of a configuration of an X-ray CT apparatus according to the embodiment;
FIG. 3 is a diagram illustrating an example of a trained model according to the first embodiment;
FIG. 4 is a diagram illustrating an example of convolution processing according to the first embodiment;
FIG. 5 is a flowchart illustrating a flow of a process performed by the image processing apparatus according to the first embodiment;
FIG. 6 is a diagram illustrating an example of processing performed by the image processing apparatus according to the first embodiment;
FIG. 7 is a diagram illustrating an example of processing performed by the image processing apparatus according to the first embodiment;
FIG. 8 is a diagram illustrating an example of a screen that is displayed by the image processing apparatus according to the first embodiment;
FIG. 9 is a diagram illustrating an example of a screen that is displayed by the image processing apparatus according to the first embodiment;
FIG. 10 is a diagram illustrating an example of a screen that is displayed by an image processing apparatus according to a modification of the first embodiment;
FIG. 11 is a diagram illustrating an example of a screen that is displayed by the image processing apparatus according to the modification of the first embodiment;
FIG. 12 is a diagram illustrating an example of a screen that is displayed by the image processing apparatus according to the modification of the first embodiment;
FIG. 13 is a diagram illustrating an example of a screen that is displayed by the image processing apparatus according to the modification of the first embodiment; and
FIG. 14 is a diagram illustrating an example of processing performed by an image processing apparatus according to a second embodiment.

### DETAILED DESCRIPTION

An X-ray apparatus according to an embodiment includes an X-ray detector and processing circuitry. The X-ray detector detects X-rays emitted from an X-ray tube and transmitted through a subject. The processing circuitry generates input data based on data obtained from the X-ray detector, when executing inference on a trained model, obtains a feature value from the trained model with respect to a region of interest set based on the input data, based on the obtained feature value, determines whether or not to continue the inference with respect to the region of interest, and stops the inference on determining not to continue the inference and continue the inference on determining to continue the inference.

An embodiment of an image processing apparatus will be described in detail below with reference to the drawings.

### First Embodiment

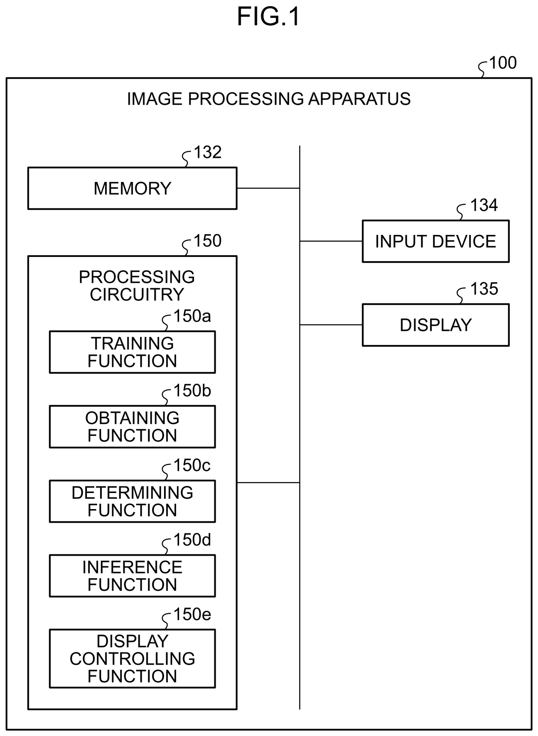

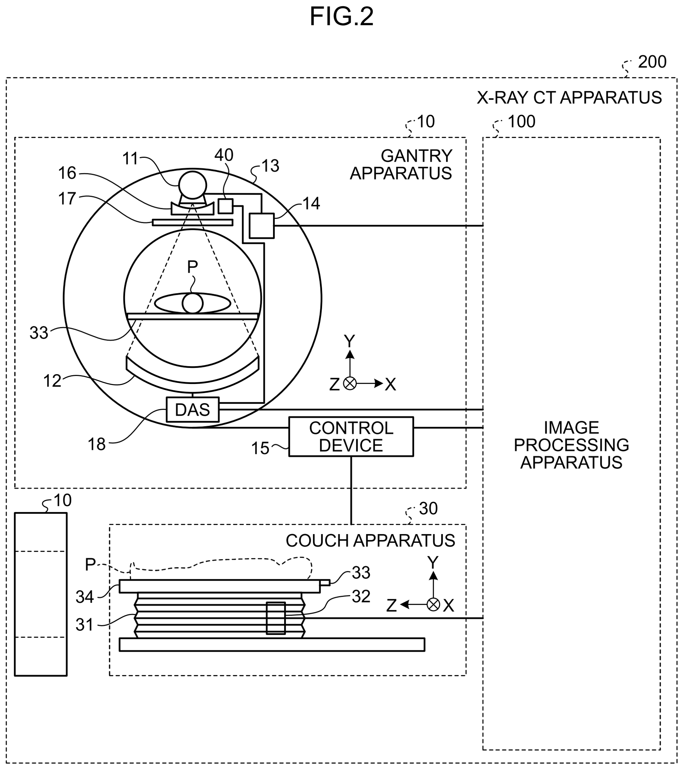

First of all, using FIG. 1 and FIG. 2, an example of a configuration of an image processing apparatus according to an embodiment will be described. FIG. 1 is a diagram illustrating an image processing apparatus 100 according to the embodiment. FIG. 2 is a diagram illustrating an example of the medical image diagnosis apparatus in which the image processing apparatus 100 according to the embodiment is incorporated. FIG. 2 illustrates the case in which the medical image diagnosis apparatus incorporating the image processing apparatus 100 is an X-ray CT apparatus 200. Note that embodiments are not limited to a case where the medical image diagnosis apparatus is an X-ray CT apparatus, and the medical image diagnosis apparatus may be, for example, another medical image diagnosis apparatus such as an ultrasound diagnosis apparatus, a magnetic resonance imaging apparatus, or a PET diagnosis apparatus. The image processing apparatus 100 according to the embodiment need not necessarily be a medial image processing apparatus, and the image processing apparatus 100 may be an image processing apparatus with a task other than medical image processing.

In the case where the image processing apparatus 100 is a medical image processing apparatus, the image processing apparatus 100 need not be incorporated in a medical image diagnosis apparatus, and the image processing apparatus 100 may function as a medical image processing apparatus independently.

In FIG. 1, the image processing apparatus 100 includes a memory 132, an input device 134, a display 135, and processing circuitry 150. The processing circuitry 150 includes a training function 150a, an obtaining function 150b, a determining function 150c, an inference function 150d, and a display controlling function 150e.

In the embodiment, respective processing functions implemented in the training function 150a, the obtaining function 150b, the determining function 150c, the inference function 150d, and the display controlling function 150e are stored in a mode of programs executable by a computer in the memory 132. The processing circuitry 150 is a processor that reads the programs from the memory 132 and executes the programs, thereby implementing the functions corresponding to the respective programs. In other words, the processing circuitry 150 in a state of having read the respective programs has the respective functions illustrated in the processing circuitry 150.

Note that FIG. 1 illustrates that the single processing circuitry 150 implements the training function 150a, the obtaining function 150b, the determining function 150c, the inference function 150d, and the display controlling function 150e; however, a plurality of independent processors may be combined to configure the processing circuitry 150 and the respective processors may execute the programs, thereby implementing the functions. In other words, each of the above-described functions may be configured as a program and the single processing circuitry 150 may execute each of the programs. In another example, a specific function may be implemented in dedicated and independent program execution circuitry. Note that, in FIG. 1, the training function 150a, the obtaining function 150b, the determining function 150c, the inference function 150d, and the display controlling function 150e are an example of a training unit, an obtaining unit, a determination unit, an execution unit (inference unit), and a display controller. Note that the display 135 is an example of a display unit.

The word "processor" used in the description above refers to, for example, a circuit such as a central processing unit (CPU), a graphical processing unit (GPU), an application specific integrated circuit (ASIC), or a programmable logic device (for example, a simple programmable logic device (SPLD), a complex programmable logic device (CPLD) or a field programmable gate array (FPGA)). The processor reads programs that are saved in the memory 132 and executes the programs, thereby implementing the functions.

Instead of saving the programs in the memory 132, the programs may be directly installed in the circuit of the processor. In this case, the processor reads the programs installed in the circuit and executes the programs, thereby implementing the functions.

By the training function 150a, the processing circuitry 150 performs machine learning and thereby generates a trained model (neural network). The generated trained model is stored in the memory 132 as required.

By the obtaining function 150b, the processing circuitry 150 obtains various sets of information from the medical image diagnosis apparatus. Using the determining function 150c and the inference function 150d, the processing circuitry 150 performs given processing to be described below. Using the display controlling function 150e, the processing circuitry 150 controls generation and display of images, and the like. For example, using the display controlling function 150e, the processing circuitry 150 causes the display 135 to display the various generated images. Additionally, using the display controlling function 150e, the processing circuitry 150 may perform overall control on the medical image diagnosis apparatus from which the image processing apparatus 100 obtains data.

The memory 132 stores the data obtained from the medical image diagnosis apparatus, image data that is generated by the processing circuitry 150, etc. For example, the memory 132 is, for example, a semiconductor memory device, such as a random access memory (RAM) or a flash memory, a hard disk, or an optical disk.

The input device 134 receives various types of instructions and information inputs from an operator. The input device 134 is, for example, a pointing device such as a mouse or a trackball, a selecting device such as a mode switch, or an input device such as a keyboard. Under the control of the processing circuitry 150 including the display controlling function 150e, the display 135 displays a graphical user interface (GUI) for receiving an input of an imaging condition, an image that is generated by the processing circuitry 150, etc. The display 135 is, for example, a display device such as a liquid crystal display device.

FIG. 2 illustrates an example of the X-ray CT apparatus 200 incorporating the image processing apparatus 100 according to the embodiment.

For example, as illustrated in FIG. 2, the X-ray CT apparatus 200 includes a gantry apparatus 10, a couch apparatus 30, and the image processing apparatus 100.

In FIG. 2, a rotational axis of a rotation frame 13 in a non-tilt state or a longitudinal direction of a couch top 33 of the couch apparatus 30 is defined as a Z-axis direction. An axial direction that is orthogonal to the Z-axis direction and that is parallel to a floor surface is defined as an X-axis direction. An axial direction that is orthogonal to the Z-axis direction and that is perpendicular to the floor surface is defined as a Y-axis direction.

The gantry apparatus 10 is an apparatus that applies X-rays to a subject P who is a patient, or the like, that detects the X-rays having been transmitted through the subject P, and that outputs the X-rays to the image processing apparatus 100. Specifically, the gantry apparatus 10 includes an X-ray tube 11, an X-ray detector 12, the rotation frame 13, an X-ray high-voltage device 14, a control device 15, a wedge 16, an X-ray aperture 17, and a data acquisition system (DAS) 18. Note that, for convenience in illustration, FIG. 2 illustrates the gantry apparatus 10 viewed in the X-axis direction and the gantry apparatus 10 viewed in the Z-axis direction; however, the X-ray CT apparatus 200 includes the single gantry apparatus 10 practically.

The X-ray tube 11 is a vacuum tube including a cathode (filament) that generates thermoelectrons and an anode (target) that generates X-rays in response to collision of the thermoelectrons. Specifically, the X-ray tube 11 applies thermoelectrons from the cathode to the anode in response to application of a high voltage from the X-ray high voltage device 14, thereby generating X-rays. For example, the X-ray tube 11 is a rotating-anode X-ray tube that generates X-rays by applying thermoelectrons to a rotating anode.

The X-ray detector 12, for example, includes a plurality of detection elements that detects the X-rays emitted from the X-ray tube 11 and having passed through the subject P and each of the detection elements outputs an electric signal corresponding to a dosage of the detected X-rays to the DAS 18. Specifically, the X-ray detector 12 includes a plurality of detection element arrays in which a plurality of detection elements are arrayed in a channel direction along an arc about a focal point of the X-ray tube 11 and has a structure in which a plurality of detection element arrays are arrayed in a slice direction (also referred to as a row direction).

For example, the X-ray detector 12 is an indirect transformation detector including a collimator, a scintillator array, and a detection element array. The collimator is arranged on a surface of the scintillator array on the side of incidence of X-rays and has an X-ray shield that absorbs scattering X-rays. For example, the collimator is a one-dimensional collimator or a two-dimensional collimator. Note that the collimator is also referred to as grid. The scintillator array is arranged on a surface of the detection element array on the side of incidence of X-rays and includes a plurality of scintillators. Each of the scintillators has a scintillator crystal that outputs light of a photon quantity corresponding to a dosage of incident X-rays. The detection element array includes a plurality of detection elements and each of the detection elements performs conversion into an electric signal corresponding to the amount of light from the scintillator. For example, the detection element array is configured by arranging a plurality of sub-arrays in which a plurality of detection elements are arrayed one-dimensionally (n rows×one column) or two-dimensionally (n rows×m columns) on the same plane in a channel direction and a slice direction in an aligned manner. For example, the detection element is a light receiving element such as a photodiode (PD) or a photo multiplier tube (PMT).

The rotation frame 13 is an annular frame that causes the X-ray tube 11 and the X-ray detector 12 to rotate on the rotational axis (a Z-axis). Specifically, in a state of supporting the X-ray tube 11 and the X-ray detector 12 such that the X-ray tube 11 and the X-ray detector 12 are opposed to each other, the rotation frame 13 is supported on a fixed frame (not illustrated in the drawings) rotatably on the rotational axis. The rotation frame 13 rotates on the rotational axis according to the control by the control device 15, thereby causing the X-ray tube 11 and the X-ray detector 12 to rotate on the rotational axis. The rotation frame 13 further supports the X-ray high-voltage device 14 and the DAS 18 in addition to the X-ray tube 11 and the X-ray detector 12.

The X-ray high-voltage device 14 includes electric circuits such as a transformer and a rectifier and includes a high-voltage generation device having a function of generating a high voltage to be applied to the X-ray tube 11 and an X-ray control device that performs control on an output voltage corresponding to the output of X-rays emitted by the X-ray tube 11. The X-ray high-voltage generation device may be a transformer type or an inverter type. The X-ray high-voltage device 14 may be provided in the rotation frame 13 or may be provided in the fixed frame (not illustrated in the drawings) that supports the rotation frame 13 rotationally in the gantry apparatus 10.

The wedge 16 is a filter for adjusting the dosage of X-rays applied from the X-ray tube 11. Specifically, the wedge 16 is a filter that transmits and attenuates X-rays that are emitted from the X-ray tube 11 such that the X-rays applied to the subject P from the X-ray tube 11 have a predetermined distribution. For example, the wedge 16 is a filter obtained by processing aluminum into a given target angle and a given thickness. The wedge 16 is also referred to as a wedge filter or a bow-tie filter.

The X-ray aperture 17 includes a lead plate for narrowing the area of radiation with X-rays having been transmitted through the wedge 16 and forms a slit by a combination of a plurality of lead plates, or the like.

The DAS 18 is processing circuitry that generates detection data based on an electric signal that is output from each detection device of the X-ray detector 12. Specifically, the DAS 18 amplifies the electric signal that is output from each detection element of the X-ray detector 12 and converts the amplified electric signal from an analog signal into a digital signal, thereby generating the detection data. The detection data generated by the DAS 18 is transmitted from a transmitter with a light emitting diode (LED) that is provided in the rotation frame 13 by optical communication to a receiver with a photodiode that is provided in a non-rotation part of the gantry apparatus 10 and is transferred to the image processing apparatus 100. In other words, the processing circuitry 150 of the image processing apparatus 150 generated the input data described above based on the data obtained from the X-ray detector 12. A method of transmitting detection data from the rotation frame 13 to the non-rotation part of the gantry apparatus 10 is not limited to optical communication, and any system may be employed as long as non-contact data transmission is enabled.

The control device 15 includes a drive mechanism, such as a motor and an actuator, and processing circuitry that controls the drive mechanism. The control device 15 has a function of, on receiving an input signal from an input interface that is attached to the image processing apparatus 100 or the gantry apparatus 10, controlling operations of the gantry apparatus 10 and the couch apparatus 30. For example, on receiving an input signal, the control device 15 performs control for causing the rotation frame 13 to rotate, control for causing the gantry apparatus 10 to tilt, and control for causing the couch apparatus 30 and the couch top 33 to operate.

The couch apparatus 30 is an apparatus on which the subject P to be scanned is laid and that moves the subject P and includes a base 31, a couch drive device 32, the couch top 33, and a support frame 34. The base 31 is a casing that supports the support frame 34 movably in the vertical direction. The couch drive device 32 is a motor or an actuator that moves the couch top 33 on which the subject P is laid in a longitudinal direction of the couch top 33. The couch top 33 that is provided on the top surface of the support frame 34 is a board on which the subject P is laid. The couch drive device 32 may move, in addition to the couch top 33, the support frame 34 in the longitudinal direction of the couch top 33.

Subsequently, the background according to the embodiment will be described.

When volume data is processed by deep learning, because of a problem of limitation of memory, and the like, in some cases, processing is not performed on the whole range of volume data and the volume data is divided into volumes of interest (VOI) in a fixed size and processing is performed on each divided VOI.

Here, for example, reducing the number of VOIs by narrowing an area of data to be processed using another trained model and then dividing the data into VOIs cam ne assumed; however, when the VOI size is large, the processing takes time in some cases.

The embodiment is made in view of the above-described background and the image processing apparatus according to the embodiment includes an obtaining unit, a determination unit, and an execution unit. When executing inference on a trained model, the obtaining unit obtains a feature value from the trained model with respect to each of regions of interest into which input data is divided. Based on the feature value obtained by the obtaining unit, the determination unit determines whether or not to continue inference with respect to each of the regions of interest. The execution unit stops inference when the determination unit determines not to continue inference and continues inference when the determination unit determines to continue inference.

As described above, terminating inference on a region of interest of which feature is less certain at the stage of extracting feature values makes it possible to reduce a calculation cost at the time of inference by a neural network and increase the speed of processing.

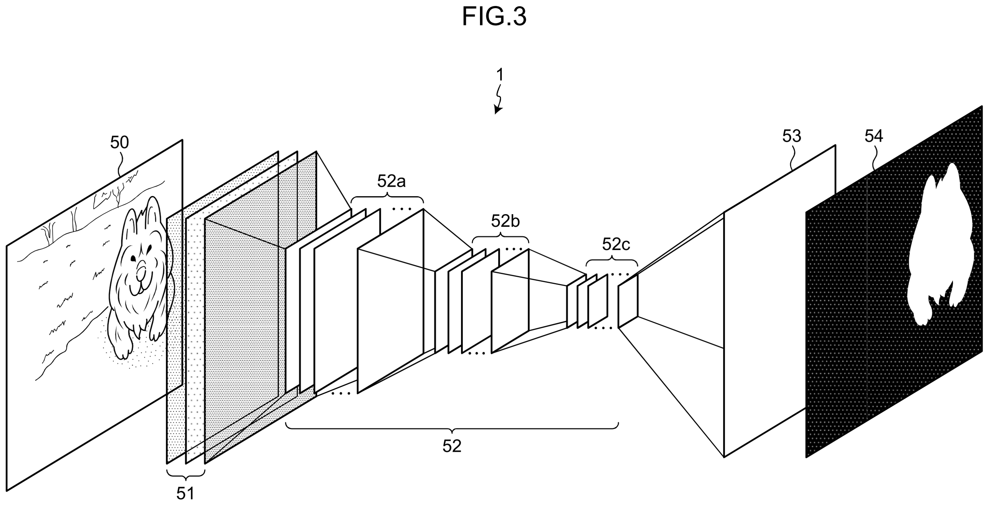

FIG. 3 illustrates an example of a trained model 1 (neural network) according to the embodiment. FIG. 3 illustrates the case where the trained model 1 (neural network) is a convolutional neural network (CNN) that detects an object. The trained model 1 (neural network) according to the embodiment however is not limited to the example illustrated in FIG. 3 and, for example, various types of neural network such as a deep neural network (DNN), a transformer, and a recursive neural network (RNN) other than a CNN may serve as the trained model according to the embodiment and usage of the neural network is not limited to object detection and the usage may be various types of usage such as area extraction, contour extraction, classification, determination on presence or absence of disease, translation using an encoder-decoder model, question answering, image/moving-image generation.

In the example illustrated in FIG. 3, a two-dimensional input image that is a color image is input to the trained model 1 as input data 50. Subsequently, the input data 50 is converted into data 51 with respect to each of channels of R, G and B.

Note that, the example in FIG. 3 describes the case where the data that is input to the trained model 1 is a two-dimensional image; however, the embodiment is not limited to this and the data that is input to the trained model 1 may be a three-dimensional image and not a color image but a bitonal image may be input as the input data 50.

The trained model 1 has an encoder part that, using convolutional processing, and the like, compresses the data 51 and thereby generates a feature value map 52 compressed compared to the data 51 and a decoder part that performs processing of a type inverse to the convolutional processing from a resultant feature value map 52c and that thereby generates data 53 in the original size again. From the data 53 that is binary data generated from the feature value map 52 via the decoder, mask data 54 on which object detection on the input data 50 is performed is generated.

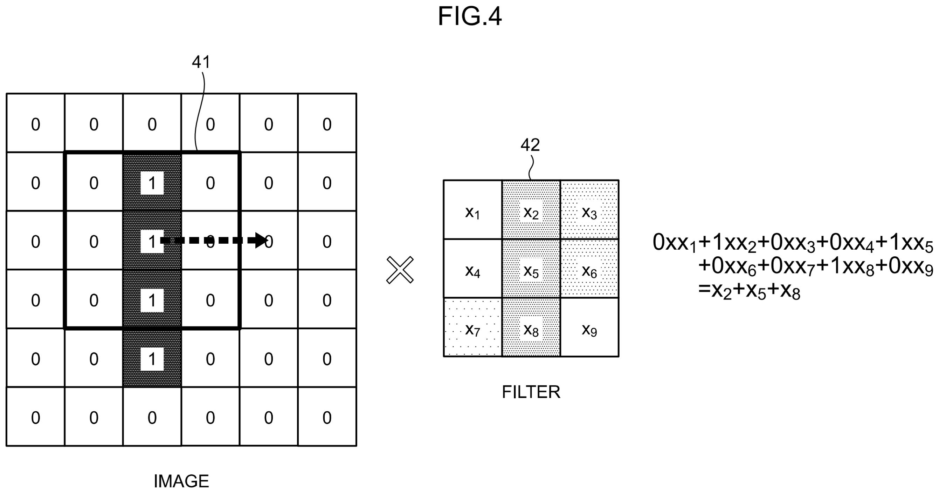

FIG. 4 illustrates an example of the convolutional processing that is executed by the encoder part of the trained model 1. As illustrated in FIG. 4, a convolutional filter 42 is applied to an area 41 in image data in each layer of the neural network, so that data to be output to the next layer is generated. The coefficient of the convolutional filter 42 is determined by training the trained model 1. As illustrated in FIG. 3, the convolutional filter 42 is applied to the data 51 in stages, so that feature value maps 52a, 52b and 52c are generated. When the feature value map 52a and the feature value map 52c are compared, the feature value map 52a has a smaller degree of data compression and has feature values in a shallow part in the neural network and, for example, tends to have low-order feature values reflecting a local structure as in edge detection. On the other hand, the feature value map 52c has a larger degree of data compression and has feature values in a deep part in the neural network and tends to have feature values of high abstraction levels capturing a more global structure.

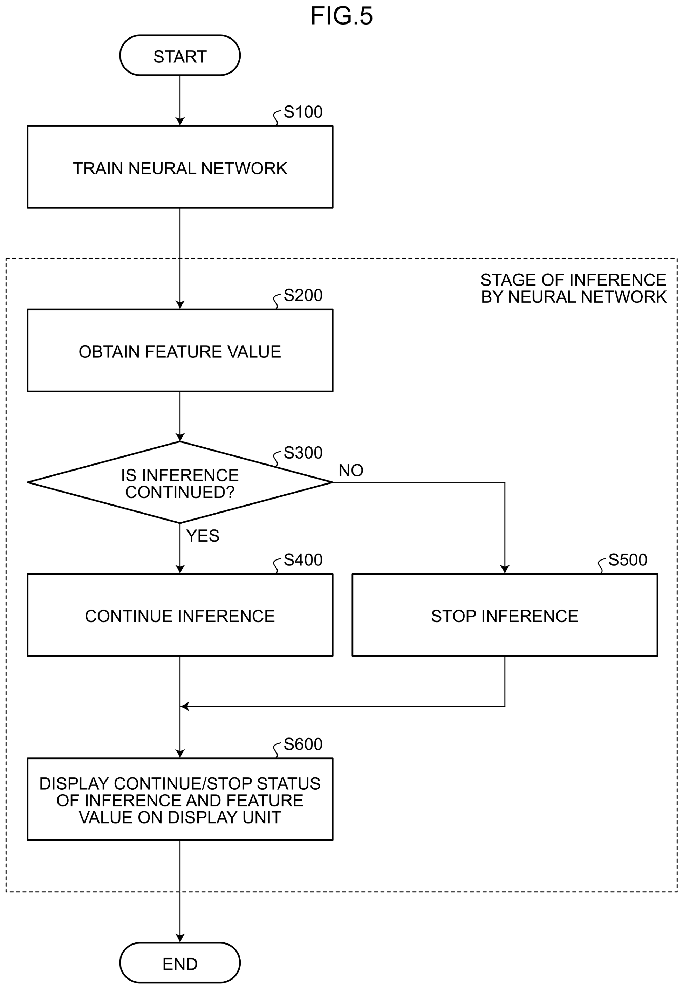

FIG. 5 is a flowchart illustrating a flow of a process performed by the image processing apparatus 100 according to the embodiment. In the flowchart in FIG. 5, step S100 is processing at the stage of training the trained model 1 and steps S200 to S600 represent processing at the stage of inference in the trained model 1. First of all, at step S100, by the training function 150a, the processing circuitry 150 performs training on the trained model 1 (neural network). Training the trained model 1 is determining a value of a parameter of the trained model 1 (neural network) and, specifically, for example, determining a coefficient of the convolutional filter 42 in each layer of the trained model 1. The trained model 1 is trained by giving a large number of pairs of the input data 50 and the mask data 54 serving as target data to the input data and updating the parameter of the trained model 1 by, for example, supervised learning. Updating the parameter in the trained model 1 is performed by calculating a gradient by backpropagation.

Subsequently, the stage of inference in the trained model 1 will be described.

The processing circuitry 150 executes the process of steps S200 to S600 with respect to each of regions of interest (ROIs) into which input data is divided. The input data, for example, may be two-dimensional image data or three-dimensional image data. The regions of interest into which the input data is divided are two-dimensional or three-dimensional regions of interest. A three-dimensional region of interest is also referred to as a voxel of interest (VOI).

At step S200, subsequently, by the obtaining function 150b, the processing circuitry 150 obtains feature values from the trained model 1. In other words, when executing inference on the trained model 1, by the obtaining function 150b, the processing circuitry 150 obtains a feature value from the trained model 1 with respect to each of the regions of interest into which the input data is divided.

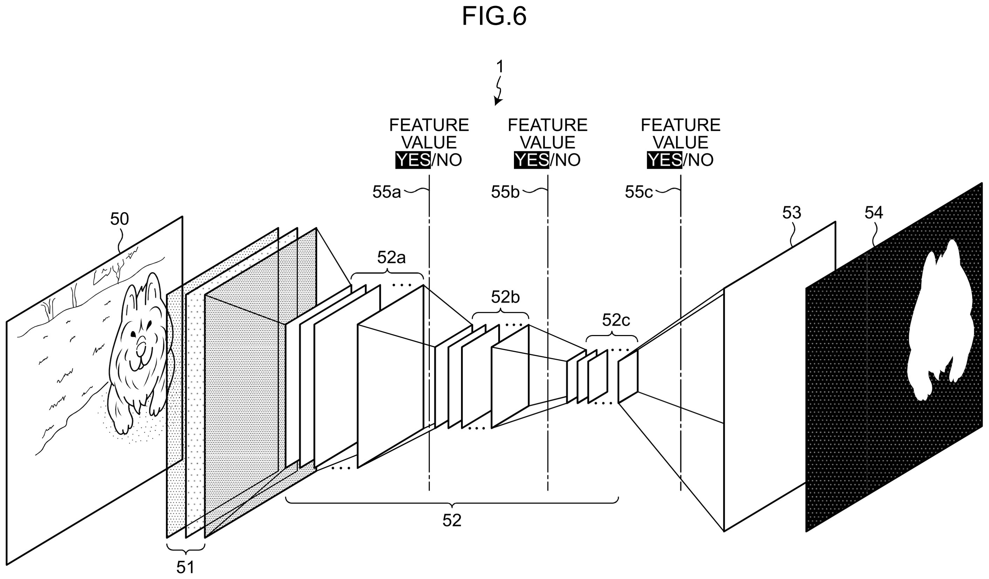

For example, as illustrated in FIG. 6, using the inference function 150d, the processing circuitry 150 generates the feature value map 52a by applying the convolutional filter 42 having a filter coefficient that is determined at the stage of training to the data 51 obtained from the input data 50 at the stage of inference, generates the feature value map 52b by further applying another convolutional filter 42 having a filter coefficient that is determined at the stage of training to the feature value map 52a, and generates the feature value map 52c by further applying another convolutional filter 42 having a filter coefficient that is determined at the stage of training to the feature value map 52b. By the obtaining function 150b, the processing circuitry 150 obtains information on these feature value maps 52a, 52b, and 52c.

Subsequently, at step S300, by the determining function 150c, the processing circuitry 150 determines, with respect to each region of interest, whether or not to continue inference on the region of interest on which processing is performed currently based on the feature value obtained by the obtaining function 150b at step S200.

When the determining function 150c determines to continue inference on the region of interest on which processing is performed currently (Yes at step S300), the process proceeds to step S400 where inference continues on the region of interest on which processing is performed currently (step S400). On the other hand, when the determining function 150c determines not to continue inference on the region of interest on which processing is performed currently (No at step S300), the process proceeds to step S500 where inference is stopped on the region of interest on which processing is performed currently (step S500).

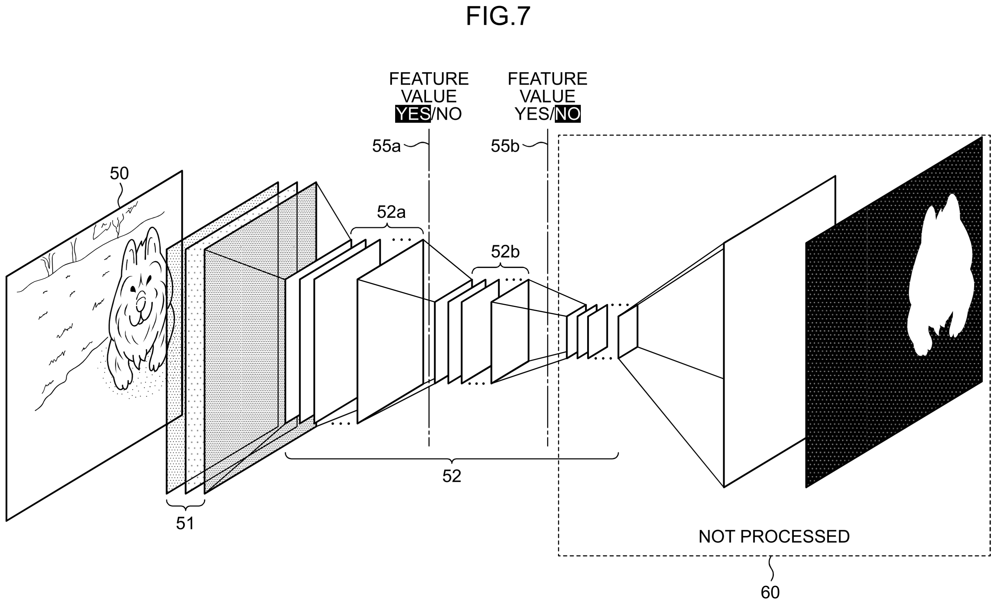

The process will be described using FIG. 6 and FIG. 7. As illustrated in FIG. 6, by the determining function 150c, the processing circuitry 150 performs determination by sequentially performing determination on the feature value in each of a plurality of layers in the trained model 1. For example, by the determining function 150c, the processing circuitry 150 sequentially performs determination on the feature value map 52a, the feature value map 52b, and the feature value map 52c in the trained model 1 and continues inference when "FEATURE VALUE YES" is determined on all determination (Yes at step S300). In determination on each of the feature value maps, for example, by the determining function 150c, the processing circuitry 150 sums all map elements contained in the feature value map and performs determination according to whether or not the sum is above a given threshold. For example, when the sum of all the map elements contained in the feature value map 52a is "10", the sum of all the map elements contained in the feature value map 52b is "20", the sum of all the map elements contained in the feature value map 52c is "30", and the given thresholds on the feature value maps 52a, 52b, and 52c are "5", "10", and "15", respectively, by the determining function 150c, the processing circuitry 150 determines FEATURE VALUE "YES" in determination areas 55a, 55b and 55c and continues inference (Yes at step S300). In this case, the process proceeds to step S400 with respect to this region of interest and, by the inference function 150d, the processing circuitry 150 executes inference and generates the data 53 and mask data 53 with respect to the region of interest.

On the other hand, for example, as illustrated in FIG. 7, when the sum of all the map elements contained in the feature value map 52a is "10", the sum of all the map elements contained in the feature value map 52b is "20", the sum of all the map elements contained in the feature value map 52c is "30", and determination thresholds on the feature value maps 52a, 52b, and 52c are "5", "30", and "15", respectively, by the determining function 150c, the processing circuitry 150 determines FEATURE VALUE "NO" with respect to the feature value map 52b because the value of the feature value map 52b is under the determination threshold and accordingly, by the determining function 150c, the processing circuitry 150 determines not to continue inference with respect to a process 60 on the current region of interest (No at step S300). In this case, with respect to the region of interest, the process proceeds to step S500 where the processing circuitry 150 stops inference.

Subsequently, at step S600, by the display controlling function 150e, the processing circuitry 150 causes the display 135 serving as a display unit to display the result of the determination at step S300.

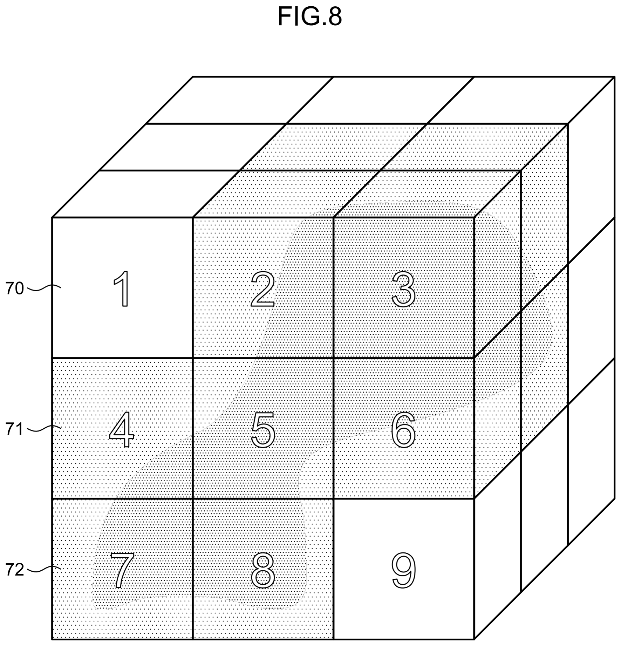

This situation is illustrated in FIG. 8. In FIG. 8, voxels 70, 71 and 72 represented by respective cubes represent voxels (VOI) of the respective regions of interest on which processing is performed by the trained model 1. The voxel 70 that is not colored represents a voxel on which, by the determining function 150c, the processing circuitry 150 determines not to continue inference at step S300. The voxels 71 and 72 that are colored represent voxels on which, by the determining function 150c, the processing circuitry 150 determines to continue inference.

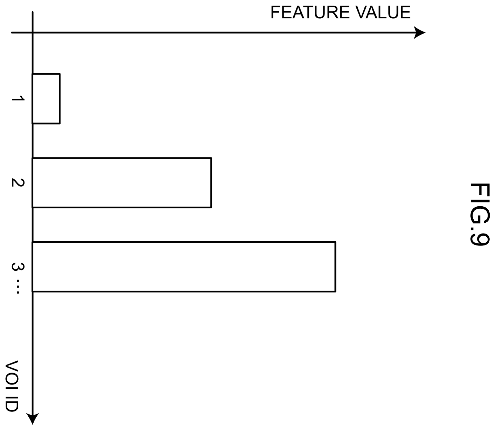

At step S300, with respect to the voxel on which, by the determining function 150c, the processing circuitry 150 determines to continue inference, by the display controlling function 150e, the processing circuitry 150 causes the display 135 serving as the display unit to display the feature value obtained by the obtaining function 150b at step S200. In an example, by the display controlling function 150e, the processing circuitry 150 may color the voxel 71 or the voxel 72 with a color corresponding to the feature value obtained by the obtaining function 150b at step S200. In an example, by the display controlling function 150e, the processing circuitry 150 colors the voxel 71 or the voxel 72 with a darker color for a larger feature value obtained by the obtaining function 150b at step S200. In another example, by the display controlling function 150e, the processing circuitry 150 may display the magnitudes of the feature values of the respective voxels, for example, by a bar chart as illustrated in FIG. 9.

In another example, by the display controlling function 150e, the processing circuitry 150 may cause the display 135 serving as the display unit to display an output of the result of inference on each region of interest such that the output is superimposed onto the result of determination on each region of interest.

By the display controlling function 150e, the processing circuitry 150 may receive an input of a change in the determination threshold according to which the determining function 150c determines whether or not to continue inference at step S300 from a user.

For example, it is known that, in a neural network, the information that a feature value map has differs according to the depth of the feature map. In an example, it is known that, as for a feature map of a shallow layer, that is, a layer close to an input image, for example, a local structure for edge extraction, or the like, is extracted. On the other hand, it is known that, as for a feature map of a deep layer, that is, a layer distant from an input image, an abstract structure over the image is extracted as the feature map. By the display controlling function 150e, the processing circuitry 150 is able to not only change the threshold according to which it is possible to determine that a feature value is extracted by changing the determination threshold but also change the "depth" according to which a feature value map is extracted mainly by changing the determination threshold according to each depth. By performing inference again based on the changed determination threshold, the processing circuitry 150 is able to increase accuracy of inference.

As described above, in the first embodiment, the determining function 150c determines whether or not to terminate inference with respect to each region of interest based on the feature value and terminates inference based on the determination. Accordingly, it is possible reduce the calculation cost at the time of inference by the neural network and increase the speed of processing at the stage of inference.

### Modification of First Embodiment

The embodiment is not limited to the above-described example and various user interfaces are assumed.

In an example, when a user performs mouse hovering over a specific region of interest or VOI and selects the specific region of interest or VOI, the display controlling function 150e may display the feature value of the region of interest such that the feature value is superimposed onto a result of the determination with respect to each region of interest. In other words, by the display controlling function 150e, the processing circuitry 150 may cause the display 135 serving as the display unit to display an output of the feature value or the result of inference such that the output is superimposed on the result of determination on each region of interest in the region of interest that is selected by the user.

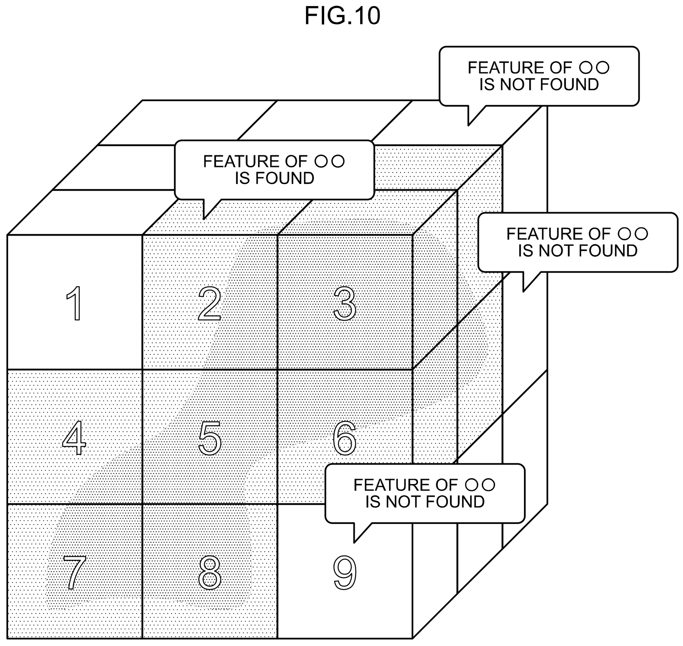

In another example, for example, as illustrated in FIG. 10, by the display controlling function 150e, the processing circuitry 150 may cause the display 135 serving as the display unit to further display the result of determination on whether or not to continue inference with respect to each region of interest in a natural language. In an example, by the display controlling function 150e, the processing circuitry 150 may display a message "FEATURE OF XX IS FOUND" on a region of interest of which feature value is above the threshold such that the message is superimposed on each voxel and may display a message "FEATURE OF XX IS NOT FOUND" on a region of interest of which feature value is under the threshold such that the message is superimposed on each voxel.

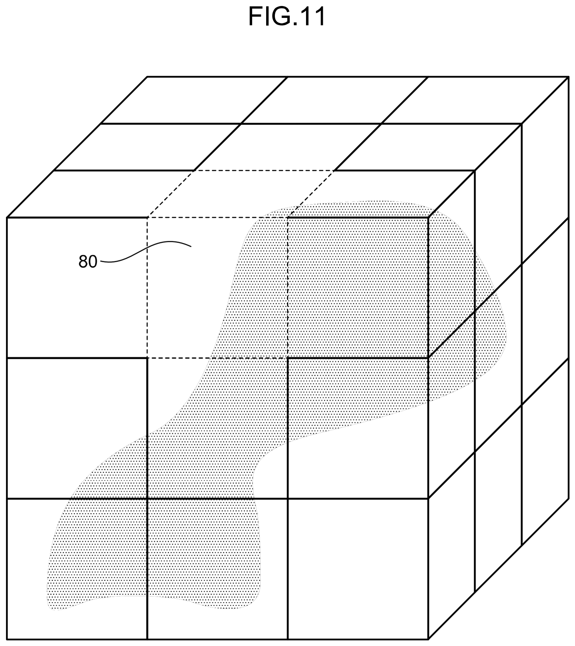

In another example, by the display controlling function 150e, the processing circuitry 150 may cause the display unit to display a region of interest on which inference is being executed in distinction from other regions of interest. In an example, for example, as illustrated in FIG. 11, by the display controlling function 150e, the processing circuitry 150 may cause the display unit to display a region of interest 80 on which inference is being executed in a blinking manner, thereby displaying the region of interest 80 in distinction from other regions of interest.

In another example, in the case where a calculation is performed by executing a plurality of algorithms (trained models), feature values obtained by neural networks corresponding to the respective algorithms may be displayed to the user. In an example, the processing circuitry 150 may obtain feature values on an algorithm basis and display the feature values in colors with darkness corresponding to the magnitudes of the obtained feature values. In an example, the display controlling function 150e may cause the display 135 to display the feature value in daker-color gradation for an algorithm with a larger feature value. It is assumed that, the more the feature value increases, the more the success rate of the algorithm increase, and thus it is assumed that an algorithm (trained model) displayed in darker-color gradation is an algorithm succeeding in the region of interest. In other words, by looking at the feature value of each algorithm, the user is able to grasp relative merits in the subject region of interest with respect to the algorithms (trained models). In other words, in the case where processing is performed by a plurality of trained models, by the display controlling function 150e, the processing circuitry 150 may cause the display unit to display information on the feature value of each of the trained models.

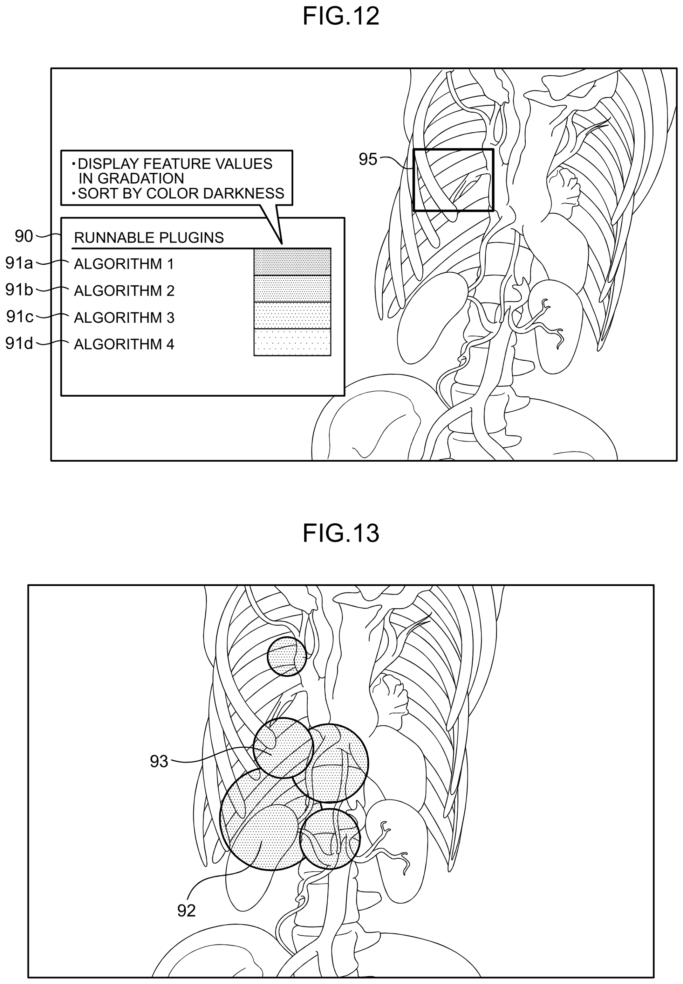

FIG. 12 illustrates the example of such a case. FIG. 12 illustrates the case where a plurality of trained models (algorithms 1 to 4) are executed on a region of interest 95. In this case, when processing is performed by the trained models (algorithms) on the given region of interest 95, by the obtaining function 150b, the processing circuitry 150 obtains a feature value in the case where inference is performed on the region of interest 95 with respect to each of the trained models. By the display controlling function 150e and using a display panel 90, the processing circuitry 150 displays information on the feature values of the respective trained models in gradation of colors in darkness corresponding to the magnitudes of the feature values and displays the information on display areas 91a, 91b, 91c and 91d while performing sorting according to the magnitude of the feature value. Accordingly, the user is able to check by sight which trained model (algorithm) has a large feature value and has superiority in the region of interest 95.

The display controlling function 150e may cause the display 135 serving as the display unit to display the feature value in each region of interest such that the feature value is superimposed onto input data. FIG. 13 illustrates an example of such an example. In the case in FIG. 13, the display controlling function 150e causes a display serving as the display unit to display feature values in the respective regions of interest in circles 92 and 93, and the like, in sizes corresponding to the magnitudes of the feature values in the corresponding regions of interest such that the feature values are superimposed onto an input medical image that is an input image. Resizing a feature value map and superimposing the feature value map onto the input image simply with respect to each region of interest as described above enables the user to check by sight the feature values in the respective regions of interest.

### Second Embodiment

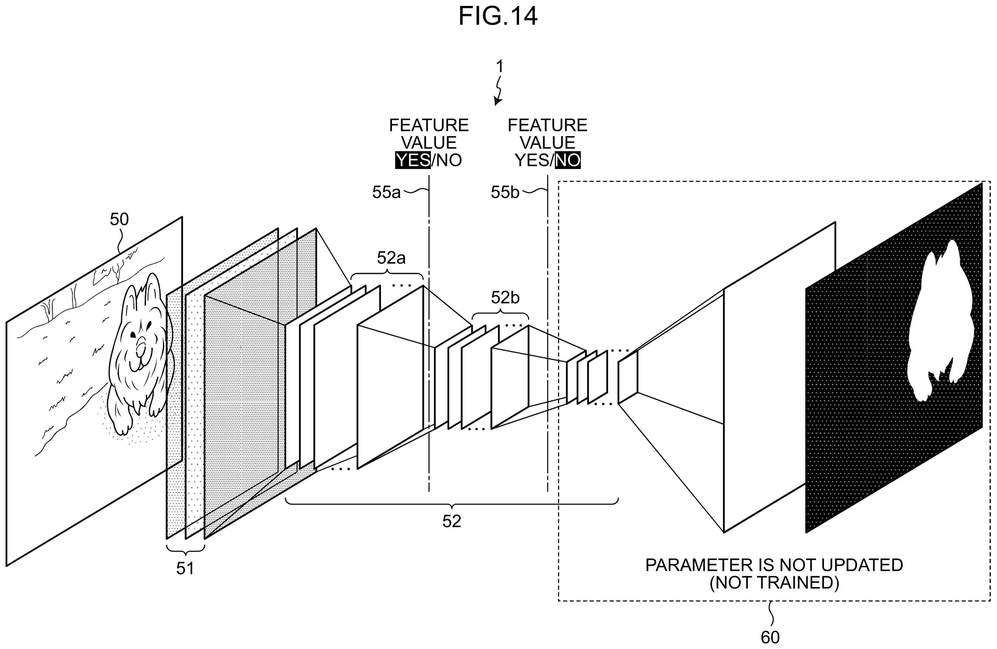

As for the first embodiment and the modification of the first embodiment, the case where inference is stopped according to the feature value with respect to each region of interest has been described. In a second embodiment, the case where, in a region of interest without feature value, training (updating a parameter) is performed on only an encoder part not at the stage of inference but at the stage of training will be described. In other words, in the second embodiment, the processing circuitry 150 includes the obtaining function 150b, the determining function 150c, and the training function 150a serving as the execution unit. When executing training on a trained model, by the obtaining function 150b, the processing circuitry 150 obtains a feature value from the trained model with respect to each of regions of interest into which input data is divided. By the determining function 150c, the processing circuitry 150 determines whether or not to continue training on the trained model in the region of interest based on the feature value obtained by the obtaining function 150b with respect to each region of interest. By the training function 150a serving as the execution unit, the processing circuitry 150 stops training with respect to the region of interest when the determining function 150c determines not to continue training with respect to the region of interest and continues training with respect to the region of interest when the determining function 150c determines to continue training. FIG. 14 illustrates such an example. For example, when the obtained feature value is under the threshold in the determination area 55b, the processing circuitry 150 does not update the parameter of a decoder part with respect to the current region of interest as illustrated in the process 60, that is, does not perform training on the decoder part of the trained model 1. This makes it possible to reduce a calculation cost required to train the trained model 1.

### Other Embodiments

In the embodiments above, various types of visualization of data have been described; however, methods of visualizing data are not limited to the above-described examples. For example, reliability of text may be visualized using probability (transition probability) that is used to select the next word in determining reliability of generated text in large language model (LLM) using Transformer, or the like. In an example, by the determining function 150c, the processing circuitry 150 determines that text formed by linkage of pairs of words with a high transition probability is reliable in comparison with text not formed as described above. The display controlling function 150e may cause the display 135 to display the text on which the determining function 150c determines high reliability, for example, in a color darker than that of the text not like the reliable text. This enables the user to check by sight high reliability of text in the LLM, which increases convenience to the user.

Furthermore, in the previous examples, the input data is divided into a plurality of regions of interest (ROIs) and the obtaining function 150b calculates feature values for each divided ROI. As one example, the input data is divided into fixed-size VOIs, and the obtaining function 150b calculates feature values for each divided VOI. However, the embodiments are not limited to this case.

For example, the input data need not be divided into fixed-size VOIs.

For example, when performing inference on a trained model, the obtaining function 150b may obtain feature values from the trained model for regions of interest set based on the input data. Furthermore, the determination function 150c determines whether to continue inference based on the acquired features. Here, the obtaining function 150b need not necessarily perform feature extraction for each region of interest, and the determination function 150c need not necessarily determine whether to continue inference for each region of interest. That is, the features calculated by the obtaining function 150b need not necessarily correspond one-to-one with the regions of interest.

For example, obtaining function 150b may calculate feature values using a trained model consisting of two stages. In this case, obtaining function 150b extracts or sets a region of interest, such as the liver region, based on the input data in the first stage of the trained model. Subsequently, the determination function 150c determines whether to continue inference for the region of interest based on the acquired features.

The region of interest may correspond to a portion of the input data or to the entire input data.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims. The accompanying claims are intended to cover such forms or modifications as would fall within the scope of the inventions as defined by the appended claims.

## Claims

1. An X-ray CT apparatus (200) comprising
an X-ray detector (12) configured to detect X-rays emitted from an X-ray tube and transmitted through a subject; and processing circuitry (150) configured to:
generate input data (50) based on data obtained from the X-ray detector (12),
when executing inference on a trained model, obtain a feature value from the trained model with respect to a region of interest set based on the input data(50),
based on the obtained feature value, determine whether or not to continue the inference with respect to the region of interest, and
stop the inference on determining not to continue the inference and continue the inference on determining to continue the inference.

2. An image processing apparatus (100) comprising processing circuitry (150) configured to:
when executing inference on a trained model, obtain a feature value from the trained model with respect to regions of interest set based on input data(50),
based on the obtained feature value, determine whether or not to continue the inference with respect to the regions of interest, and
stop the inference on determining not to continue the inference and continue the inference on determining to continue the inference.

3. The image processing apparatus (100) according to claim 2, wherein the regions of interest are regions of interest into which the input data(50) is divided.

4. The image processing apparatus (100) according to claim 2, wherein the regions of interest are regions of interest that correspond to a portion of the input data(50).

5. The image processing apparatus (100) according to claim 2, wherein the processing circuitry (150) is configured to
obtain feature values for each of the regions of interest into which the input data(50) is divided, and
determine whether or not to continue the inference with respect to each of the regions of interest based on the obtained feature values.

6. The image processing apparatus (100) according to claim 2, wherein the processing circuitry (150) includes a display control unit that causes a display (135) to display a result of determination.

7. The image processing apparatus (100) according to any one of claims 2 to 6, wherein the input data(50) is two-dimensional or three-dimensional image data, and
the regions of interest are two-dimensional or three-dimensional regions of interest.

8. The image processing apparatus (100) according to any one of claims 2 to 6, wherein the processing circuitry (150) is configured to perform the determination by sequentially performing determination on the feature value in each of a plurality of layers in the trained model.

9. The image processing apparatus (100) according to claim 6, wherein, as for the regions of interest that are selected by a user, the processing circuitry (150) causes the display (135) to display an output of the feature value or a result of the inference such that the output is superimposed onto a result of the determination with respect to each of the regions of interest.

10. The image processing apparatus (100) according to claim 6, wherein the processing circuitry (150) receives an input of a change in a threshold of the determination from a user.

11. The image processing apparatus (100) according to claim 6, wherein the processing circuitry (150) causes the display (135) to further display a result of the determination in a natural language.

12. The image processing apparatus (100) according to claim 6, wherein the processing circuitry (150) causes the display (135) to display a region of interest on which the inference is being executed in distinction from other regions of interest.

13. The image processing apparatus (100) according to claim 6, wherein the trained model includes a plurality of trained models, and
the processing circuitry (150) causes the display (135) to display information on the feature values of the respective trained models.

14. The image processing apparatus (100) according to claim 6, wherein the processing circuitry (150) causes the display (135) to display the feature value such that the feature value is superimposed onto input data(50).

15. An image processing apparatus (100) comprising processing circuitry (150) configured to:
when executing training on a trained model, obtain a feature value from the trained model with respect to a region of interest set based on input data(50),
based on the obtained feature value, determine whether or not to continue training on the trained model with respect to the region of interest,
stop the training with respect to the region of interest on determining not to continue the training with respect to the region of interest and continue the training with respect to the region of interest on determining to continue the training.
